# EUROPÄISCHE PATENTSCHRIFT

(11) **EP 0 864 563 B1**
(45) Veröffentlichungstag und Bekanntmachung des Hinweises auf die Patenterteilung: **02.10.2002**
(21) Anmeldenummer: 98103683.3
(22) Anmeldetag: 03.03.1998
(51) Int. Cl.: C07D 207/16, A61K 7/48, C07C 233/47, C07C 229/04

(54) **N-Acyl-hydroxyaminosäuureester und ihre Verwendung zum Schutz von Haut und Haar**
N-Acyl-hydroxy amino acid esters used to protect skin and hair
Esters d'aminoacid N-acylés pour la protection de la peau et des cheveaux

(30) Priorität: 14.03.1997 DE 19710612
(43) Veröffentlichungstag der Anmeldung: 16.09.1998
(73) Patentinhaber: HAARMANN & REIMER GMBH, D-37601 Holzminden (DE)
(72) Erfinder: Ley, Jakob, Dr., 37603 Holzminden (DE); Langner, Roland, 37639 Bevern (DE)
(74) Vertreter: Petrovicki, Wolfgang, Dr.

(56) Entgegenhaltungen:
- DE-C- 4 420 736
- GB-A- 1 246 141
- US-A- 4 016 287
- FEIL ET AL: "Stability of 3-glycosyloxyprolines in an alkaline medium. Synthesis of model compounds." CARBOHYDR. RES., Bd. 31, 1973, Seiten 311-322, XP002068588

## Beschreibung

Die vorliegende Erfindung betrifft N-Acyl-hydroxyaminosäureester, ein Verfahren zu ihrer Herstellung, als Zwischenprodukte für die Herstellung dieser N-Acyl-hydroxyaminosäureester verwendbare Hydroxyaminosäureester und deren Salze, die Verwendung von N-Acyl-hydroxyaminosäureestern zur Herstellung von Hautpflegemitteln und Hautpflegemittel mit einem Gehalt an N-Acyl-hydroxyaminosäureester. Hautpflegemittel im Sinne der Erfindung sind kosmetische Produkte zum Schutz und zur Pflege von Haut und Haar.

Die oberste menschliche Hautschicht, die Hornschicht oder das Stratum Corneum besteht aus abgestorbenen Corneozyten, die durch Lipide zusammengehalten werden. Diese Lipide setzen sich aus ca. 50 Gew.-% Ceramiden, etwa 15 Gew.-% Cholesterin, 5 Gew.-% Cholesterylestern und 33 Gew.-% freien Fettsäuren (K. De Paepe et al., SÖFW-Journal 1996, 122 (4), 199-200, 202-204) zusammen. Ceramide sind lipophile Amide langkettiger Fettsäuren, die sich im Allgemeinen vom Sphingosin oder Phytosphingosin ableiten. Die Lipidschicht und insbesondere die Ceramide spielen eine wichtige Rolle als wasserrückhaltende Barriere für die Haut und sind somit für die Feuchtigkeitsspeicherung in der Hornschicht großenteils verantwortlich. Diese Funktion wird durch die Fähigkeit der Ceramide zur Bildung von Doppelmembranschichten, zwischen denen Wasser eingelagert wird, erklärt. Die externe Applikation von Ceramiden führt zur Wiederherstellung der Lipidbarriere, so dass Hautschädigungen, die durch Zerstörung derselben verursacht werden, repariert werden können (R.D. Petersen, Cosm. Toil. 1992, 107 (2), 45-49).

Da Ceramide weder synthetisch noch aus natürlichen Quellen gut zugänglich sind, ist ihre Verfügbarkeit begrenzt. Es hat daher bereits Versuche gegeben, ceramidähnliche Strukturen oder synthetische Barrierelipide ("synthetic barrier lipids") zu synthetisieren und zur Hautpflege einzusetzen (G. Imokawa et al., J. Soc. Cosmet. Chem. 1989, 40, 273-285; S. Watkins et al, SÖFW-Journal 1995, 121 (4), 228-238).

So werden beispielsweise in der DE-PS 44 03 258 Alkylamide der Struktur 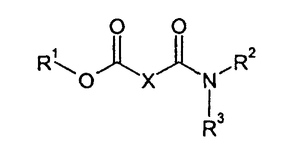 vorgeschlagen, worin
- R¹: für einen verzweigten Alkyl- und/oder Alkenylrest mit 12 bis 50 Kohlenstoffatomen, R² für Wasserstoff oder einen gegebenenfalls hydroxysubstituierten Alkylrest mit 1 bis 30 Kohlenstoffatomen, R³ für einen Hydroxyalkylrest mit 2 bis 12 Kohlenstoffatomen und 1 bis 10 Hydroxylgruppen oder einen Glycosylrest und X für einen linearen oder verzweigten Alkylenrest mit 1 bis 6 Kohlenstoffatomen steht.

Aus WO 94/07 844 sind Amide der Struktur 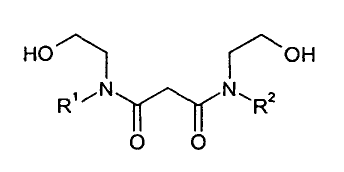 bekannt, wobei R¹ und R² Kohlenwasserstoffreste darstellen.

Für die Hautpflege werden in der DE-PS 44 20 736 Zucker- und Aminosäurederivate der Formel 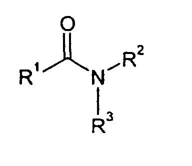 vorgeschlagen, worin
- R¹CO: für einen Hydroxyacylrest mit 3 bis 8 Kohlenstoffatomen und 2 bis 7 Hydroxygruppen steht und
- R² und R³: gleich oder verschieden sein können und für Wasserstoff, eine lineare oder verzweigte Alkylgruppe mit 1 bis 50 Kohlenstoffatomen, oder gemeinsam eine Gruppe mit der Formel

-R⁴-COO-R⁵
worin
- R⁴: für eine Alkylengruppe mit 1 bis 18 Kohlenstoffatomen, die eine weitere Estergruppe COO-R⁶, worin R⁶ eine lineare oder verzweigte Alkylgruppe mit 1 bis 50 Kohlenstoffatomen ist, enthalten kann, steht und R⁵ eine lineare oder verzweigte Alkylgruppe mit 6 bis 50 Kohlenstoffatomen ist mit der Maßgabe, dass mindestens zwei der Gruppen R², R³, R⁵ und R⁶ langkettige Reste mit 6 bis 50 Kohlenstoffatomen darstellen oder mindestens eine der Gruppen R², R³, R⁵ und R⁶ eine verzweigte Alkylgruppe ist, wobei die Verzweigungen mindestens 6 Kohlenstoffatome aufweisen.

In Carbohydrate Research, 1973, Seiten 311 bis 322 wird der N-Acetyl-3-hydroxyprolinethylester und in GB-A 1 246 141 der N-Acetyl-4-hydroxyprolinethylester beschrieben. Allerdings werden weder längerkettige Derivate, die sich potentiell in die Hautlipide einlagern könnten, noch irgendwelche Threoninderivate beschrieben.

In US-A 4016 287 werden dermatologische Kompositionen zur Hautpflege, enthaltend verschiedene N-Acylaminosäurealkylester beschrieben. Dabei ist entweder der Acylrest längerkettig (10 bis 22 C-Atome) und der Alkylrest kurzkettig (1 bis 3 C-Atome) oder umgekehrt. Zudem dürfen laut der Beschreibung ausschließlich unpolare Aminosäuren wie z.B. Methionin, Alanin, Valin, Glycin, β-Alanin, Leucin, Isoleucin, Phenylalanin, Sarcosin oder 4-Aminobuttersäure als Aminosäuren eingesetzt werden. Es ist nicht naheliegend, dass die polareren Hydroxyaminosäurederivate einen hautpflegenden Effekt verursachen können.

Trotz dieser Versuche ist der Erfolg, der mit diesen Stoffen erreichbar ist, nicht befriedigend.

Die Aufgabe der vorliegenden Erfindung bestand darin, neue biomimetische Verbindungen mit ceramidähnlicher Wirkung zu entwickeln, die einfach und aus natürlichen Rohstoffen herzustellen sind.

Gegenstand der Erfindung sind N-Acyl-hydroxyaminosäureester der Formel 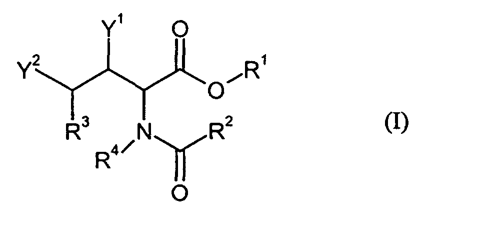 worin
- R¹: eine lineare, verzweigte oder cyclische Alkyl- oder Alkenylgruppe mit 5 bis 50 Kohlenstoffatomen,
- R²: eine linerare oder verzweigte Alkyl- oder Alkenylgruppe mit 1 bis 49 Kohlenstoffatomen,
- Y¹ und Y²: unabhängig voneinander Wasserstoff oder Hydroxyl bedeuten,
- R³ und R⁴: zusammen für die Alkylenreste -CH₂-, -CH₂-CH₂-, -CH(OH)-, -CH(OH)-CH₂- oder -CH₂-CH(OH)- stehen und zusammen mit der Kette zwischen R³ und R⁴ einen 5 oder 6-gliedrigen heterocyclischen Ring bilden.

Insbesondere bevorzugt sind N-Acyl-hydroxyaminosäureester (I), worin
- R³ und R⁴: zusammen eine -CH₂-Gruppe darstellen und zusammen mit der Kette zwischen R³ und R⁴ einen 5-gliedrigen heterocyclischen Ring bilden und einer der beiden Reste Y¹ und Y² einen Hydroxylrest und der andere ein Wasserstoffatom darstellt (N-Acyl-hydroxyprolinester).
oder Verbindungen (I') (Siehe Anspruch 2) wo
- R¹: eine lineare Alkyl- oder Alkenylgruppe mit 5 bis 24 Kohlenstoffatomen und
- R²: eine lineare Alkyl- oder Alkenylgruppe mit 2 bis 23 Kohlenstoffatomen bedeuten und
und
- R³ und R⁴: Wasserstoffatome sind und Y¹ einen Hydroxylrest und Y² ein Wasserstoffatom darstellen (N-Acyl-threoninalkylester),

Die im Sinne der Erfindung eingesetzten N-Acyl-hydroxyaminosäureester stärken die natürliche Barrierefunktion der Haut und vermitteln einen überraschend positiven hautglättenden und pflegenden Eindruck.

Die erfindungsgemäßen N-Acyl-hydroxyaminosäureester sind farblose bzw. schwach elfenbeinartig gefärbte, geruchlose Substanzen, die sich homogen in die Ölphase von kosmetischen Mitteln einarbeiten lassen.

Besonders bevorzugt sind N-Acyl-threoninester und N-Acyl-hydroxyprolinester, worin R¹ einen unverzweigten Alkyl- oder Alkenylrest mit 5 bis 24 Kohlenstoffatomen und R² einen unverzweigten Alkyl- oder Alkenylrest mit 2 bis 23 Kohlenstoffatomen bedeuten, wodurch ein guter Einbau der amphiphilen Verbindungen in die Doppelmembran der Lipidbarriere und damit eine Verbesserung der Hauteigenschaften erreicht wird.

Ein weiterer Gegenstand der Erfindung betrifft ein Verfahren zur Herstellung der N-Acylhydroxyaminosäureester (I),
wonach man
a) Hydroxyaminosäuren der Formel 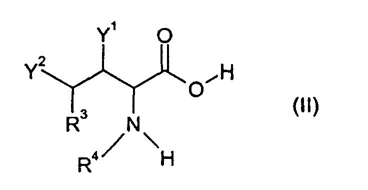 worin
   - R³, R⁴, Y¹ und Y²: die oben angegebene Bedeutung haben, oder deren kationische oder anionische Salze
   mit verzweigten oder geradkettigen Alkoholen der Formel

   R¹-OH (III)

   worin
   R¹ die oben angegebene Bedeutung hat, verestert, und
b) die resultierenden Zwischenprodukte der Formel 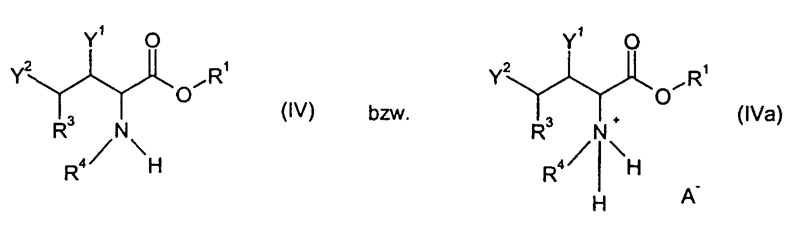 in denen
   - R¹, R³, R⁴, Y¹ und Y²: die oben angegebene Bedeutung haben und A- ein anorganisches oder organisches Anion, z.B. Halogenid-, Sulfat-, Phosphat-, Acetat- oder p-Toluolsulfonatanion, darstellt,
   mit Verbindungen der Formel

   R²-COX¹ (V),
worin
- R²: die oben angegebene Bedeutung hat und
- X¹: eine Hydroxylgruppe, ein Halogenatom, eine heterocyclische Gruppe (gegebenenfalls mit annelliertem C₅-C₁₂-Aromat) mit 5 bis 6 Ringatomen, von denen bis zu 3 Heteroatome sein können (bis zu 1 Schwefel-, bis zu 2 Sauerstoff- und bis zu 3 Stickstoffatome), C₆-C₁₂-Arylsulfonyl, eine Gruppe -O-X² oder -S- X², worin X² eine C₁-C₁₀-Alkyl- oder C₂-C₁₀-Alkenylgruppe, eine C₆-C₁₂-Arylgruppe, eine heterocyclische Gruppe (wie oben), C₆-C₁₂-Arylsulfonyl, eine Aminogruppe oder eine Gruppe R²-CO- bedeutet, bevorzugt eine Hydroxygruppe, ein Halogenatom oder eine Gruppe -O-X² oder -S-X², worin X² eine nitrosubstituierte C₆-C₁₂-Arylgruppe, C₆-C₁₂-Arylsulfonyl oder eine cyclische sekundäre C₄-C₁₀-Aminogruppe bedeutet, insbesondere aber eine Hydroxygruppe, Chlor, Brom, eine Gruppe -O-X² oder -S- X², wobei X² p- oder o-Nitrophenyl, p-Toluolsulfonyl, N-Benzotriazolyl oder N-Succinimidyl, darstellt, kondensiert.

Als Ausgangsverbindungen zur Herstellung der erfindungsgemäßen N-Acyl-hydroxyaminosäureester sind die Hydroxyaminosäuren (II), bevorzugt die natürlich vorkommenden oder synthetischen Threonine und Hydroxyproline (L-, D- oder D,L-, in Form aller Diastereomere) geeignet. Insbesondere werden das in der Natur weitverbreitete L-Threonin und das in nativem Kollagen und auch in Pflanzen vorkommende trans-4-Hydroxy-L-prolin verwendet.

Als Alkohole (III) kommen insbesondere lineare primäre, verzweigte primäre und sekundäre cycloaliphatische Alkohole in Betracht. Beispiele für lineare Alkohole sind die nativen Fettalkohole auf pflanzlicher Basis, wie z.B. Laurylalkohol, Myristylalkohol, Cetylalkohol, Stearylalkohol, Behenylalkohol, Oleylalkohol, Elaidylalkohol, Ricinolalkohol, Linoleylalkohol, Linolenylalkohol sowie deren technische Gemische. Beispiele für sekundäre cycloaliphatische Alkohole sind die natürlich vorkommenden oder synthetischen Alkohole (-)-Menthol oder (+)-Menthol.

Die Synthese der Zwischenprodukte (IV) bzw. (IVa) kann durch Veresterung der obengenannten Hydroxyaminosäuren (II) mit den obengenannten Alkoholen (III) in an sich bekannter Weise im Alkohol (III) mit oder ohne Lösungsmittel durch Zugabe einer kondensationsfördernden Verbindung durchgeführt werden. Die Reaktion wird bevorzugt im Alkohol (III) unter Zugabe von trockenem Chlorwasserstoffgas oder Thionylchlorid bei bevorzugt 10 bis 150°C durchgeführt. Typische Reaktionszeiten liegen zwischen 0,5 und 24 Stunden. Alternativ kann die Reaktion in einem organischen Lösungsmittel, das mit Wasser ein Azeotrop bildet, unter Zugabe einer Säure durchgeführt werden. Dabei werden bevorzugt aromatische Lösungsmittel, insbesondere Benzol, Toluol oder Xylol, unter Zugabe von starken Säuren, insbesondere Schwefelsäure und aromatischen Sulfonsäuren, in der Siedehitze verwendet und das Reaktionswasser azeotrop entfernt.

Neu ist insbesondere die Veresterung von Hydroxyprolinen und Threoninen mit primären Alkoholen mit 5 oder mehr Kohlenstoffatomen und die Isolierung und Reinigung der Verbindungen (IV) bzw. (IVa) durch Kristallisieren, Umkristallisieren z.B. aus niederen Ketonen oder durch Flüssigkeitschromatographie.

Überraschenderweise wurde gefunden, dass die Zwischenprodukte (IV) bzw. (IVa), vor allem die Hydroxyprolinester und deren Ammoniumsalze, eine antimikrobielle Wirkung gegen hautbesiedelnde Bakterien und Pilze zeigen, insbesondere gegen Corynebacterium-, Aspergillus-, Pseudomonas- und Staphylococcus-Spezies, insbesondere gegen Corynebacterium xerosis, Aspergillus niger, Pseudomonas aeruginosa und Staphylococcus aureus.

Als Acylierungsmittel (V) sind insbesondere lineare und verzweigte primäre aktivierte Carbonsäurederivate geeignet. Bevorzugt werden Fettsäurehalogenide oder die Aktivester von Fettsäuren, insbesondere die o- oder p-Nitrophenyl-Ester oder die Ester des N-Hydroxysuccinimids, die alle in bekannter Weise aus den Carbonsäuren durch Halogenierung oder Veresterung hergestellt werden können, verwendet. Beispiele für lineare primäre Carbonsäuren sind die nativen Fettsäuren Caprinsäure, Laurinsäure, Myristinsäure, Palmitinsäure, Stearinsäure, Arachinsäure, Behensäure, Erucasäure, Palmitoleinsäure, Ölsäure, Petroselinsäure, Elaidinsäure, Linolsäure, α-Linolensäure, γ-Linolensäure und Arachidonsäure sowie deren technische Gemische und die nativen Hydroxyfettsäuren Cerebronsäure (2-Hydroxytetracosansäure), α-Hydroxynervonsäure (2-Hydroxy-15-Z-tetracosensäure) und Ricinolsäure.

Die crfindungsgemäßen N-Acyl-hydroxyaminosäureester (I) können durch Umsetzung der Zwischenprodukte (IV) bzw. (IVa) mit einem Acylierungsmittel (V) hergestellt werden. Bevorzugt wird die Reaktion in einem Lösungsmittel unter Zugabe von Säurefängern durchgeführt. Insbesondere wird die Acylierung in Wasser oder einem Wasser/Lösungsmittelgemisch mit Fettsäurehalogeniden oder Fettsäureestern des N-Hydroxysuccinimids in Gegenwart eines Säurefangers oder einer Base durchgeführt. Als Säurefänger oder Base können Metallcarbonate und Metallhydrogencarbonate der 1. bis 3. Hauptgruppe, wie z.B. Natriumhydrogencarbonat, tertiäre Amine, wie z.B. Triethylamin, oder Heterocyclen, wie z.B. Pyridin, eingesetzt werden. Im Anschluß können die Produkte durch Destillation, Umkristallisieren, beispielsweise aus niederen Alkoholen, oder Flüssigkeitschromatographie gereinigt werden.

Die N-Acyl-hydroxyaminosäureester (I) können in Hautpflegemitteln in Mengen von 0,1 bis 50, vorzugsweise von 0,1 bis 10, insbesondere von 0,1 bis 5 Gew.-% - bezogen auf das Gesamtgewicht der Formulierung - enthalten sein und dabei sowohl als "Wasser-in-Öl" als auch "Öl-in-Wasser"-Emulsionen vorliegen; weitere übliche Hilfs- und Zusatzstoffe können in Mengen von 5 bis 95, vorzugsweise 10 bis 95 Gew.-% - bezogen auf das Gesamtgewicht der Formulierung - enthalten sein. Femer können die Formulierungen Wasser in einer Menge bis zu 99 Gew.-%, vorzugsweise 5 bis 90 Gew.-% - bezogen auf das Gesamtgewicht der Formulierung - aufweisen.

In einer bevorzugten Ausführungsform der Erfindung können die erfindungsgemäßen N-Acyl-hydroxyaminosäureester mit Ceramiden, weiteren Fettsäureamiden, Cholesterin, Cholesterinfettsäureestern, Fettsäuren, Triglyceriden, Cerebrosiden, Phospholipiden und ähnlichen Stoffen gemischt werden, wobei Liposomen entstehen können.

In einer weiteren Ausführungsform können die erfindungsgemäßen N-Acyl-hydroxyaminosäureester schließlich auch in Squalen oder Squalan gelöst und gegebenenfalls mit anderen Inhaltsstoffen zusammen mit flüchtigen oder nichtflüchtigen Siliconverbindungen als wasserfreie oder fast wasserfreie Systeme formuliert werden.

Die erfindungsgemäß als synthetische Barrierelipide verwendbaren N-Acyl-hydroxyaminosäureester tragen zur Stärkung der natürlichen Barrierefunktion der Haut gegenüber Umwelteinflüssen und äußeren Reizen bei. Sie verbessern die Geschmeidigkeit und Elastizität von Haut und Haaren, tragen zur Steigerung des Feuchtigkeitsgehalts bei und schützen Haut und Haare vor Austrocknung.

Ein weiterer Gegenstand der Erfindung betrifft daher die Verwendung der N-Acyl-hydroxyaminosäureester (I) als synthetische Barrierelipide zur Herstellung von Hautpflegemitteln im engeren Sinn, in denen sie in Mengen von 0,1 bis 50, vorzugsweise 0,1 bis 10, insbesondere von 0,1 bis 5 Gew.-% - bezogen auf das Gesamtgewicht der Hautpflegemittel - und zur Herstellung von Haarpflegemitteln, in denen sie in Mengen von 0,1 bis 15, vorzugsweise von 0,1 bis 5, insbesondere von 0,1 bis 2 Gew.-% - bezogen auf das Gesamtgewicht der Haarpflegemittel - enthalten sein können. Typische Beispiele sind Hautcremes, Softcremes, Nährcremes, Sonnenschutzcremes, Nachtcremes, Hautöle, Pflegelotionen, Körperaerosole, Haarshampoo, Haarspülung, Haarkur, Haarspray oder Haarwasser.

### Beispiele

### trans-4-Hydroxy-L-prolin-(1-dodecyl)esterhydrochlorid

In 1-Dodecanol (190 g, 1,02 mol) wurde bei 25 bis 30°C Thionylchlorid (15,5 g, 130 mmol) unter Rühren zugegeben. Nach Hinzufügen von trans-4-Hydroxy-Lprolin (15 g, 114,4 mmol) wurde die Suspension 5 Stunden bei 100°C gerührt. Von der nun klaren Reaktionslösung wurde das 1-Dodecanol im Vakuum abdestilliert und der abgekühlte kristalline Rückstand in Methanol gelöst und filtriert. Das Lösungsmittel wurde im Vakuum abdestilliert und die Destillation bei beginnender Kristallisation abgebrochen. Der Rückstand wurde mit Diethylether gefällt, der Niederschlag abfiltriert und mit Diethylether gewaschen. Ausbeute: 35,3 g (105,1 mmol, 92 % d.Th.) weißes Kristallpulver. ¹H-NMR (CD₃SOCD₃) δ = 0,86 (3H, t), 1,25 (18H, m), 1,61 (2H, m), 2,08 (1H, m), 2,18 (1H, m), 3,06 bis 3,3 (teilweise unter H₂O-Signal, m), 4,16 (2H, m) 4,45 (2H, m), 5,53 (1H, m), 9,5 bis 10 ppm (2H, breit); ¹³C-NMR (CD₃SOCD₃) δ = 168,8 (s), 68,4 (d), 65,8 (t), 57,4 (d), 53,2 (d), 37,0 (t), 31,2 (t), 28,94 (t), 28,90 (t), 28,86 (t), 28,81 (t), 28,60 (t), 28,51 (t), 27,8 (t), 25,08 (t), 22,0 (t), 13,9 ppm (q). MS (ESI) m/z = 300,1 (M⁺ des Kations).

In analoger Weise wurden die folgenden Verbindungen als weiße oder bernsteinfarbene Kristallpulver erhalten:
trans-4-Hydroxy-L-prolin-(1-hexyl)esterhydrochlorid
trans-4-Hydroxy-L-prolin-(1-heptyl)esterhydrochlorid
trans-4-Hydroxy-L-prolin-(1-octyl)esterhydrochlorid
trans-4-Hydroxy-L-prolin-(1-nonyl)esterhydrochlorid
trans-4-Hydroxy-L-prolin-(1-decyl)esterhydrochlorid
trans-4-Hydroxy-L-prolin-(1-tetradecyl)esterhydrochlorid
trans-4-Hydroxy-L-prolin-(1-hexadecyl)esterhydrochlorid.

### L-Threonindodecylester

L-Threonin (2,6 g, 21,8 mmol), 1-Dodecanol (5,18 g, 27,23 mmol) und p-Toluolsulfonsäuremonohydrat (4,10 g, 22 mmol) wurden in Benzol (80 ml) an einem Dean-Stark-Wasserabscheider 16 Stunden zum Rückfluß erhitzt. Nach dem Verdünnen mit 100 ml Toluol wurde die organische Phase mit Wasser und ges. wässriger Kochsalzlösung gewaschen, über Na₂SO₄ getrocknet, filtriert und das Filtrat im Vakuum eingedampft. Das farblose Öl wurde bei 0,2 mbar 1 Stunde getrocknet. Ausbeute: 8 g. ¹H-NMR (CDCl₃) δ = 0,87 (3H, t), 1,24 (3H,d), 1,24 bis 1,40 (18H, m), 1,60 bis 1,70 (2H, m), 3,25 (1H, d), 3,84 (1H, m), 4,13 ppm (2H, m); ¹³C-NMR (CDCl₃) δ = 174,2 (s), 68,3 (d), 65,5 (t), 60,0 (d), 31,9 (t), 29,7 (2C, t), 29,6 (t), 29,5 (t), 29,4 (t), 29,2 (t), 28,6 (t), 25,9 (t), 22,7 (t), 19,8 (q), 14,1 ppm (q).

In analoger Weise wurde die folgende Verbindung als farbloses Öl erhalten: L-Threonindecylester.

### N-(1-Hexadecanoyl)-trans-4-hydroxy-L-prolin-(1-dodecyl)ester

trans-4-Hydroxy-L-prolindodecylesterhydrochlorid (10 g, 29,8 mmol) wurde in 1,4-Dioxan (125 ml) und Wasser (125 ml) gelöst und unter Rühren zuerst Natriumhydrogencarbonat (10,4 g, 123,8 mmol) und anschließend 1-Hexadecanoylchlorid (9 ml, 8,19 g, 29,8 mmol) zugegeben. Die Reaktionsmischung wurde 1 Stunde bei Raumtemperatur gerührt. Die Suspension wurde auf pH 1 bis 1,5 eingestellt und anschließend filtriert. Der Rückstand wurde mit Wasser neutral gewaschen und im Vakuum in der Schmelze getrocknet. Ausbeute: 15,1 g (28 mmol, 94 % d.Th., 97 bis 100 % Reinheit [HPLC]) weiße wachsartige Kristallmasse, Smp.: 39 bis 44°C. ¹H-NMR (CDCl₃) δ = 0,89 (6H, t), 1,2 bis 1,4 (40H, m), 1,5 bis 1,7 (6H, m), 2,05 bis 2,40 (4H, m), 3,51 (1H, dm), 3,79 (1H, dd), 4,12 (2H, m), 4,5 bis 4,6 ppm (2H, m); ¹³C-NMR (CDCl₃) δ = 172,50 (s), 172.48 (s), 70,4 (d), 65,4 (t), 57,6 (d), 55,1 (t), 37,9 (t), 34,6 (t), 32,0, 29,72 (2C), 29,70 (2C), 29,68 (4C), 29,66, 29,62, 29,56 (2C), 29,48, 29,40, 29,38 (2C), 29,28, 28,56, 25,85, 24,68, 22,70 (2C), 14,1 (2C, q,q). Elementaranalyse: ber.: C 73,7 %, H 11,8 %, N 2,6 %, O 11,9 %; gef.: C 73,5 %, H 11,8 %, N 2,5 %, O 12,2 %.

In analoger Weise wurden die folgenden Verbindungen in Form von farblosen Ölen oder farblosen wachsartigen Substanzen erhalten:
N-(1-Decanoyl)-trans-4-hydroxy-L-prolin-(1-dodecyl)ester : Smp. <20°C
N-(1-Dodecanoyl)-trans-4-hydroxy-L-prolin-(1-dodecyl)ester: Smp. 20-25°C
N-(1-Tetradecanoyl)-trans-4-hydroxy-L-prolin-(1-dodecyl)ester: Smp. 32-39°C
N-(1-Tetradecanoyl)-trans-4-hydroxy-L-prolin-(1-tetradecyl)ester : Smp. 40-45°C
N-(1-Dodecanoyl)-trans-4-hydroxy-L-prolin-(1-tetradecyl)ester : Smp. 35-40°C
N-(1-Octadecanoyl)-trans-4-hydroxy-L-prolin-(1-hexadecyl)ester. : Smp. 40-50°C

### N -Dodecanoyl-L-threonindodecylester

Der L-Threonindodecylester (8,8 g) wurde in 1,4-Dioxan (60ml) und Wasser (50 ml) gelöst und Natriumhydrogencarbonat (3 g, 35,7 mmol) sowie Laurinsäure-(N-succinimidyl)ester (6,48 g, 21,8 mmol) zugegeben. Die trübe Mischung wurde bei ca. 90°C eine Stunde unter Rückfluß gekocht und anschließend abkühlen gelassen. Bei 50°C wurde die Mischung mit 10 % HCl auf pH 2-3 eingestellt und im Eisbad auf 15 bis 20°C gekühlt. Bei ca. 40°C beginnt das Produkt auszufallen. Das Rohprodukt wird abfiltriert, mit Wasser gewaschen und aus Methanol umkristallisiert. Ausbeute: 7,75 g (16,5 mmol, 78 % über 2 Stufen), farblose Kristallnadeln (HPLC >99 %). Smp.: 97-98°C, ¹H-NMR (CDCl₃) δ = 0,86 bis 0,90 (6H, m), 1,23 (3H, d, 6,4 Hz), 1,24 bis 134 (34H, m), 1,60 bis 1,70 (4H, m), 2,00 (1H, d, 5,8 Hz), 2,43 (2H, dd, 2*7,4 Hz), 4,16 (2H, t, 6,8 Hz), 4,30 bis 4,37 (1H, m), 4,61 (1H, dd, 8,8 Hz, 2,6 Hz), 6,15 ppm (1H, d, 8,8 Hz); ¹³C-NMR (CDCl₃) δ = 173,7 (s), 171,3 (s), 68,3 (d), 65,9 (t), 57,0 (d), 36,7 (t), 31,9 (2C, t), 29,7 (2C, t), 29,6 (5C, t), 29,5 (t), 29,4 (2C, t), 29,3 (t), 29,2 (t), 28,5 (t), 25,8 (t), 25,7 (t), 22,7 (2C, t), 20,0 (q), 14,1 ppm (2C, q); Elementaranalyse: ber.: C 71,6 %, H 11,8 %, N 3,0 %, O 13,6 %; gef.: C 71,8 %, H 11,2 %, N 2,9 %, O 13,5 %.

In analoger Weise wurde die folgende Verbindung in Form von farblosen wachsartigen Kristallen erhalten:
N-(1-Decanoyl)-L-threonin-(1-decyl)ester.

| **Formulierungsbeispiel "Öl-in-Wasser"-Emulsion** | | | |
|---|---|---|---|
| **Teil** | **Rohstoffname (Hersteller)** | **Chemische Bezeichnung** | **Gehalt in** **Gew.-%** |
| A | Arlatone 983 S® (ICI) | Ether von Polyethylenglycol mit Glycerylmonostearat | 1,20 |
| | Brij 76® (ICI) | 3,6,9,12,15,18,21,24,27,30,33, 36-Decaoxaoctatetracontan-1-ol | 1,20 |
| | Cutina MD® (Henkel KGaA) | Glycerylmonostearat | 3,50 |
| | Baysiloneöl M10® (Bayer) | Polydimethylsiloxan | 0,80 |
| | Eutanol G® (Henkel KGaA) | 2-Octyldodecanol | 3,00 |
| | Paraffinöl 65cp(Henry Lamotte) | Mineralöl | 8,00 |
| | N-(1-Hexadecanoyl)-trans-4-hydroxy-L-prolin-(1-dodecyl)ester | | 1,00 |
| | | | |
| B | Wasser, dest. | | 51,30 |
| | Phenonip® (Nipa Laboratorien GmbH) | 2-Phenoxyethanol und 4-Hydroxybenzoesäuremethylester und 4-Hydroxybenzoesäureethylester und 4-Hydroxybenzoesäurepropylester und 4-Hydroxybenzoesäurebutylester | 0,50 |
| | Propylenglycol | | 2,00 |
| | | | |
| C | Wasser, dest. | | 25,00 |
| | Carbopol 2050® (B.F. Goodrich Chemical) | Carbomer (CA-Bezeichnung) | 0,40 |
| | | | |
| | wässrige Natriumhydroxidlösung, 10 % | | 1,90 |
| | | | |
| D | Parfumöl | | 0,20 |
| Teil A wird auf 90°C erhitzt. Teil B wird auf 90°C erhitzt und zu Teil A gegeben. Teil C: Carbopol wird in Wasser quellen gelassen und neutralisiert. Teil C wird bei ca. 60°C zu Teil A/B gegeben. Teil D wird zu Teil A/B/C bei Raumtemperatur gegeben. | | | |

## Patentansprüche

1. N-Acyl-hydroxyaminosäureester der Formel 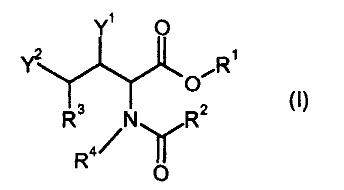 worin
R¹ eine lineare, verzweigte oder cyclische Alkyl- oder Alkenylgruppe mit 5 bis 50 Kohlenstoffatomen,
R² eine lineare oder verzweigte Alkyl- oder Alkenylgruppe mit 1 bis 49 Kohlenstoffatomen,
Y¹ und Y² unabhängig voneinander Wasserstoff oder Hydroxyl bedeuten,
R³ und R⁴ zusammen für die Alkylenreste -CH₂-, -CH₂-CH₂-, -CH(OH)-, -CH(OH)-CH₂- oder -CH₂-CH(OH)- stehen und zusammen mit der Kette zwischen R³ und R⁴ einen 5 oder 6-gliedrigen, heterocyclischen Ring bilden.

2. N-Acyl-hydroxyaminosäureester der Formel 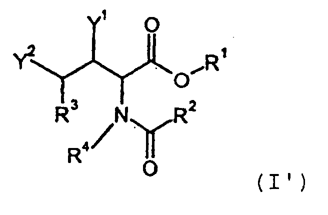 worin
R¹ eine lineare Alkyl- oder Alkenylgruppe mit 5 bis 24 Kohlenstoffatomen
und
R² eine lineare Alkyl- oder Alkenylgruppe mit 2 bis 23 Kohlenstoffatomen bedeuten und
R³ und R⁴ Wasserstoffatome sind und Y¹ einen Hydroxylrest und Y² ein Wasserstoffatom darstellen.

3. N-Acyl-hydroxyaminosäureester nach Anspruch 1, worin
R¹ eine lineare Alkyl- oder Alkenylgruppe mit 5 bis 24 Kohlenstoffatomen, und
R² eine lineare Alkyl- oder Alkenylgruppe mit 2 bis 23 Kohlenstoffatomen bedeuten,
und
R³ und R⁴ zusammen eine -CH₂-Gruppe darstellen und zusammen mit der Kette zwischen R³ und R⁴ einen 5-gliedrigen, heterocyclischen Ring bilden und einer der beiden Reste Y¹ und Y² einen Hydroxylrest und der andere ein Wasserstoffatom darstellt.

4. Verfahren zur Herstellung von N-Acyl-hydroxyaminosäureestern nach den Ansprüchen 1 bis 3, **dadurch gekennzeichnet, dass**
a) Hydroxyaminosäuren der Formel 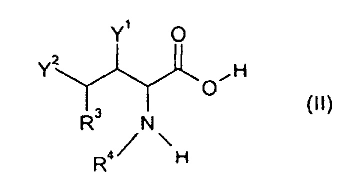 worin
R³, R⁴, Y¹ und Y² die oben angegebene Bedeutung haben, oder deren kationische oder anionische Salze
mit verzweigten oder geradkettigen Alkoholen der Formel
R¹-OH (III)
worin
R¹ die oben angegebene Bedeutung hat, verestert, und
b) die resultierenden Zwischenprodukte der Formel 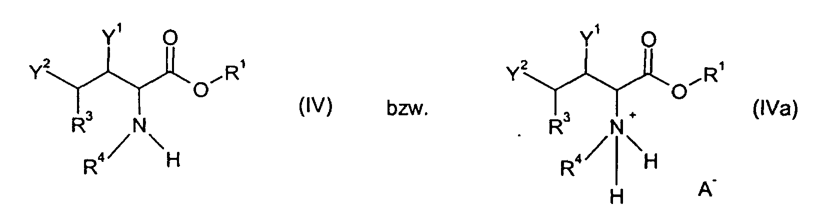 in denen
R¹, R³, R⁴, Y¹ und Y² die oben angegebene Bedeutung haben und A- ein anorganisches oder organisches Anion darstellt,
mit Verbindungen der Formel
R²-COX¹ (V),
worin
R² die oben angegebene Bedeutung hat und
X¹ eine Hydroxylgruppe, ein Halogenatom, eine heterocyclische Gruppe (gegebenenfalls mit annelliertem C₅-C₁₂-Aromat) mit 5 bis 6 Ringatomen, von denen bis zu 3 Heteroatome sein können (bis zu 1 Schwefel-, bis zu 2 Sauerstoff- und bis zu 3 Stickstoffatome), C₆-C₁₂-Arylsulfonyl, eine Gruppe -O-X² oder -S- X², worin X² eine C₁-C₁₀-Alkyl- oder C₂-C₁₀-Alkenylgruppe, eine C₆-C₁₂-Arylgruppe, eine heterocyclische Gruppe (wie oben), C₆-C₁₂-Arylsulfonyl, eine Aminogruppe oder eine Gruppe R²-CO-, darstellt, kondensiert.

5. Verfahren nach Anspruch 4, **dadurch gekennzeichnet, dass** man als Aminosäuren vorkommende oder synthetische Hydroxyproline einsetzt.

6. Verfahren nach den Ansprüchen 4 und 5, **dadurch gekennzeichnet, dass** man als Alkohole lineare primäre, verzweigte primäre oder sekundäre cyclische Alkohole einsetzt.

7. Verfahren nach den Ansprüchen 4 bis 6, **dadurch gekennzeichnet, dass** man als aktivierte Säurederivate lineare oder verzweigte primäre Carbonsäurehalogenide einsetzt.

8. Verfahren nach den Ansprüchen 4 bis 6, **dadurch gekennzeichnet, dass** man als aktivierte Säurederivate lineare oder verzweigte primäre Carbonsäureester des N-Hydroxysuccinimids oder des o- oder p-Nitrophenols einsetzt.

9. Verbindungen der Formeln 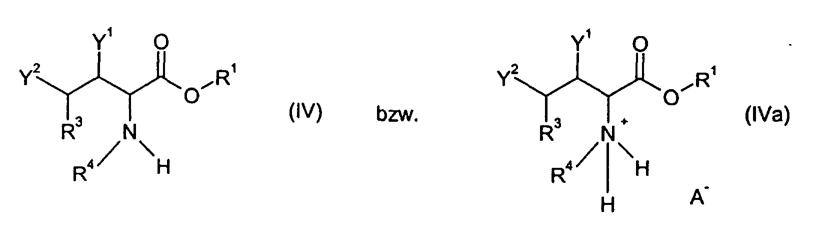 in denen
R¹, R³, R⁴, Y¹ und Y² die in Anspruch 1 genannte Bedeutung haben
und
A- ein anorganisches oder organisches Anion darstellt.

10. Verbindungen nach Anspruch 9, **dadurch gekennzeichnet, dass** in den Formeln IV und IVa
R¹, R³, R⁴, Y¹ und Y² die in Anspruch 2 genannte Bedeutung haben.

11. Verbindungen nach Anspruch 9, **dadurch gekennzeichnet, dass** in den Formeln IV und IVa
R¹, R³, R⁴, Y¹ und Y² die in Anspruch 3 genannte Bedeutung haben.

12. Verwendung von N-Acyl-hydroxyaminosäureestern nach den Ansprüchen 1 bis 3 zur Herstellung von kosmetischen Produkten zum Schutz und zur Pflege von Haut und Haar.

13. Kosmetische Produkte mit einem Gehalt an N-Acyl-hydroxyaminosäureester gemäß den Ansprüchen 1 bis 3.

14. Verwendung der Verbindungen nach den Ansprüchen 9 bis 11 als Wirkstoffe gegen hautbesiedelnde Bakterien oder Pilze.

## Claims

1. N-Acyl-hydroxyamino acid esters of the formula 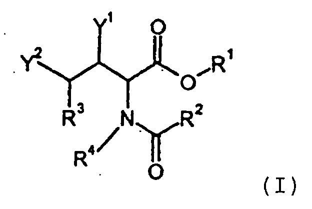 in which
R¹ means a linear, branched or cyclic alkyl or alkenyl group with 5 to 50 carbon atoms,
R² means a linear or branched alkyl or alkenyl group with 1 to 49 carbon atoms,
Y¹ and Y² mutually independently mean hydrogen or hydroxyl,
R³ and R⁴ together denote the alkylene residues, -CH₂-, -CH₂-CH₂-, -CH(OH)-, -CH(OH)-CH₂- or -CH₂-CH(OH)- and, together with the chain between R³ and R⁴, form a 5- or 6-membered, heterocyclic ring.

2. N-Acyl-hydroxyamino acid esters of the formula 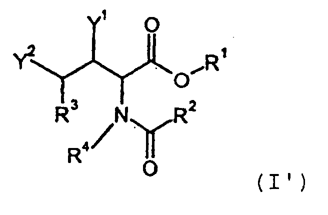 in which
R¹ means a linear alkyl or alkenyl group with 5 to 24 carbon atoms
and
R² means a linear alkyl or alkenyl group with 2 to 23 carbon atoms and
R³ and R⁴ are hydrogen atoms and Y¹ represents a hydroxyl residue and Y² a hydrogen atom.

3. N-Acyl-hydroxyamino acid esters according to claim 1, in which
R¹ means a linear alkyl or alkenyl group with 5 to 24 carbon atoms and
R² means a linear alkyl or alkenyl group with 2 to 23 carbon atoms,
and
R³ and R⁴ together represent a -CH₂- group and, together with the chain between R³ and R⁴, form a 5-membered heterocyclic ring and one of the two residues Y¹ and Y² represents a hydroxyl residue and the other a hydrogen atom.

4. A process for the production of N-acyl-hydroxyamino acid esters according to claims 1 to 3, **characterised in that**
a) hydroxyamino acids of the formula 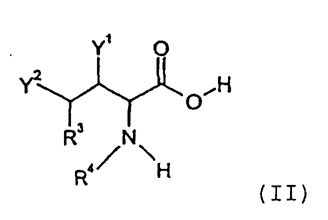 in which
R³, R⁴, Y¹ and Y² have the above-stated meaning, or the cationic or anionic salts thereof
are esterified with branched or linear alcohols of the formula
R¹-OH (III)
in which
R¹ has the above-stated meaning, and
b) the resultant intermediates of the formula 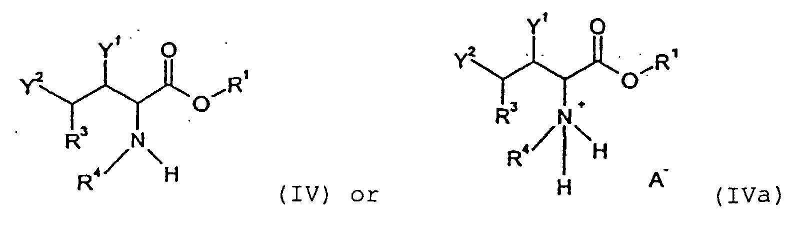 in which
R¹, R³, R⁴, Y¹ and Y² have the above-stated meaning and A⁻ represents an inorganic or organic anion,
are condensed with compounds of the formula
R²-COX¹ (V),
in which
R² has the above-state meaning and
X¹ represents a hydroxyl group, a halogen atom, a heterocyclic group (optionally with a fused C₅-C₁₂ aromatic) with 5 to 6 ring atoms, up to 3 of which may be heteroatoms (up to 1 sulfur atom, up to 2 oxygen atoms and up to 3 nitrogen atoms), C₆-C₁₂ arylsulfonyl, a group -O-X² or -S-X², in which X² represents a C₁-C₁₀ alkyl or C₂-C₁₀ alkenyl group, a C₆-C₁₂ aryl group, a heterocyclic group (as above), C₆-C₁₂ arylsulfonyl, an amino group or a group R²-CO-.

5. A process according to claim 4, **characterised in that** hydroxyprolines which occur as amino acids or synthetic hydroxyprolines are used as the amino acids.

6. A process according to claims 4 and 5, **characterised in that** the alcohols used are linear primary, branched primary or secondary cyclic alcohols.

7. A process according to claims 4 to 6, **characterised in that** the activated acid derivatives used are linear or branched primary carboxylic acid halides.

8. A process according to claims 4 to 6, **characterised in that** the activated acid derivatives used are linear or branched primary carboxylic acid esters of N-hydroxysuccinimide or of o- or p-nitrophenol.

9. Compounds of the formulae 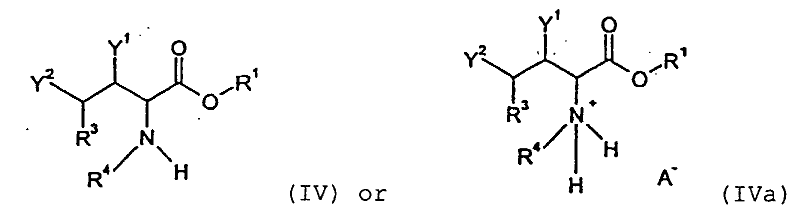 in which
R¹, R³, R⁴, Y¹ and Y² have the meaning stated in claim 1
and
A⁻ represents an inorganic or organic anion.

10. Compounds according to claim 9, **characterised in that**, in the formulae IV and IVa,
R¹, R³, R⁴, Y¹ and Y² have the meaning stated in claim 2.

11. Compounds according to claim 9, **characterised in that**, in the formulae IV and IVa,
R¹, R³, R⁴, Y¹ and Y² have the meaning stated in claim 3.

12. Use of N-acyl-hydroxyamino acid esters according to claims 1 to 3 for the production of cosmetic products for protecting and caring for the skin and hair.

13. Cosmetic products containing N-acyl-hydroxyamino acid esters according to claims 1 to 3.

14. Use of the compounds according to claims 9 to 11 as substances active against skin-colonising bacteria or fungi.

## Revendications

1. Esters de N-acyl-hydroxyaminoacides de formule 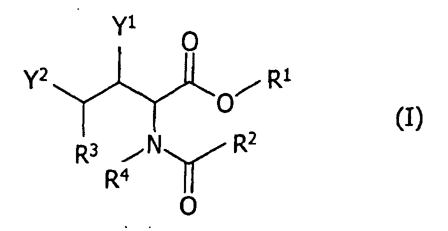 où
R¹ représente un groupe alkyle ou alcényle linéaire, ramifié ou cyclique ayant 5 à 50 atomes de carbone,
R² représente un groupe alkyle ou alcényle linéaire ou ramifié ayant 1 à 49 atomes de carbone,
Y¹ et Y² représentent indépendamment l'un de l'autre l'hydrogène ou hydroxyle,
R³ et R⁴ représentent ensemble les restes alkylène -CH₂-, -CH₂-CH₂-, -CH(OH)-, -CH(OH)-CH₂- ou -CH₂-CH(OH)- et forment avec la chaîne entre R³ et R⁴ un cycle hétérocyclique à 5 ou 6 chaînons.

2. Esters de N-acyl-hydroxyaminoacides de formule 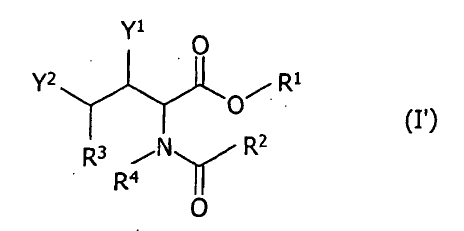 où
R¹ représente un groupe alkyle ou alcényle linéaire ayant 5 à 24 atomes de carbone,
R² représente un groupe alkyle ou alcényle linéaire ayant 2 à 23 atomes de carbone, et
R³ et R⁴ sont des atomes d'hydrogène et Y¹ représente un reste hydroxyle et Y² représente un atome d'hydrogène.

3. Esters de N-acyl-hydroxyaminoacides selon la revendication 1, où
R¹ représente un groupe alkyle en alcényle linéaire ayant 5 à 24 atomes de carbone et
R² représente un groupe alkyle ou alcényle linéaire ayant 2 à 23 atomes de carbone,
et
R³ et R⁴ représentent ensemble un groupe -CH₂- et forment avec la chaîne entre R³ et R⁴ un cycle hétérocyclique à 5 chaînons et l'un des deux restes Y¹ et Y² représente un reste hydroxyle et l'autre représente un atome d'hydrogène.

4. Procédé de production des esters de N-acyl-hydroxyaminoacides (I),
selon lequel
a) on estérifie des hydroxyaminoacides de formule 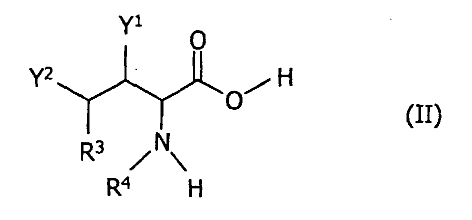 où R³, R⁴, Y¹ et Y² ont la signification indiquée ci-dessus, ou leurs sels cationiques ou anioniques,
avec des alcools ramifiés ou linéaires de formule
R¹-OH (III)
où
R¹ a la signification indiquée ci-dessus, et
b) on condense les produits intermédiaires résultants de formule 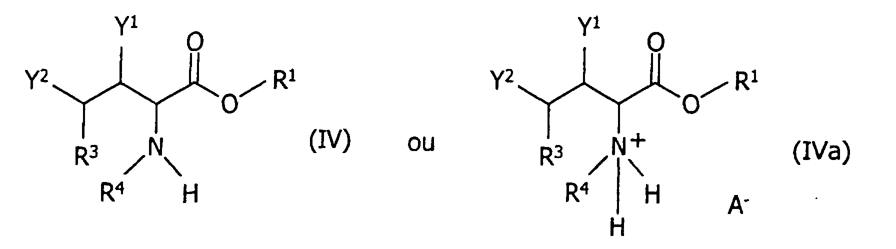
où
R¹, R³, R⁴, Y¹ et Y² ont la signification indiquée ci-dessus et A⁻ représente un anion inorganique ou organique, par exemple un anion halogénure, sulfate, phosphate, acétate ou p-toluènesulfonate, avec des composés de formule
R²-COX¹ (V)
où
R² a la signification indiquée ci-dessus et
X¹ représente un groupe hydroxyle, un atome d'halogène, un groupe hétérocyclique (éventuellement avec un groupe aromatique en C₁-C₁₂ condensé) ayant 5 à 6 atomes cycliques, parmi lesquels jusqu'à 3 atomes peuvent être des hétéroatomes (jusqu'à 1 atome de soufre, jusqu'à
2 atomes d'oxygène et jusqu'à 3 atomes d'azote) arylsulfonyle en C₆-C₁₂, un groupe -O-X² ou S-X², où X² représente un groupe alkyle en C₁-C₁₀ ou un groupe alcényle en C₂-C₁₀, un groupe aryle en C₆-C₁₂, un groupe hétérocyclique (comme ci-dessus), arylsulfonyle en C₆-C₁₂, un groupe amino ou un groupe R²-CO-.

5. Procédé selon la revendication 4, **caractérisé en ce que** l'on utilise comme aminoacides des hydroxyprolines naturelles ou synthétiques.

6. Procédé selon les revendications 4 et 5, **caractérisé en ce que** l'on utilise comme alcools des alcools primaires linéaires, primaires ramifiés ou cycliques secondaires.

7. Procédé selon les revendications 4 à 6, **caractérisé en ce que** l'on utilise comme dérivés d'acides activés des halogénures d'acides carboxyliques primaires linéaires ou ramifiés.

8. Procédé selon les revendications 4 à 6, **caractérisé en ce que** l'on utilise comme dérivés d'acides activés des esters d'acides carboxyliques primaires linéaires ou ramifiés du N-hydrosuccinimide ou du o- ou p-nitrophénol.

9. Composés des formules 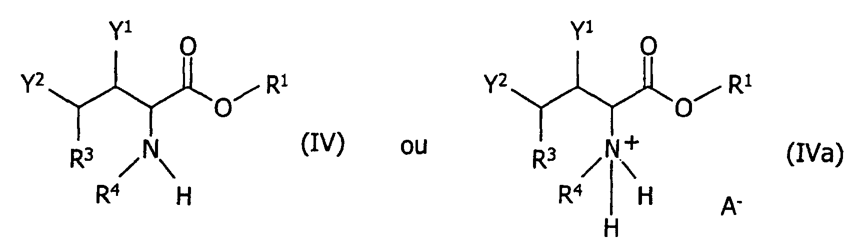 où
R¹, R³, R⁴, Y¹ et Y² ont la signification citée dans la revendication 1,
et
A⁻ représente un anion inorganique ou organique.

10. Composés selon la revendication 9, **caractérisés en ce que**, dans les formules IV et IVa, R¹, R³, R⁴, Y¹ et Y² ont la signification citée dans la revendication 2.

11. Composés selon la revendication 9, **caractérisés en ce que**, dans les formules IV et IVa, R¹, R³, R⁴, Y¹ et Y² ont la signification citée dans la revendication 3.

12. Utilisation d'esters de N-acyl-hydroxyaminoacides selon les revendications 1 à 3 pour la fabrication de produits cosmétiques pour la protection et pour les soins de la peau et des cheveux.

13. Produits cosmétiques ayant une teneur en esters de N-acyl-hydroxyaminoacides selon les revendications 1 à 3.

14. Utilisation des composés selon les revendications 9 à 11 comme principes actifs contre les bactéries ou champignons colonisant la peau.
